# EUROPEAN PATENT APPLICATION

(11) **EP 2 746 382 A1**
(43) Date of publication of application: **25.06.2014**
(21) Application number: 11870985.6
(22) Date of filing: 15.08.2011
(51) Int. Cl.: C12M 1/00, C12M 1/04, C12M 1/06

(54) **VORTEX BIOREACTOR**

(71) Applicant: Obshchestvo S Organichennoi Otvetstvennostyu "Tsentr Vykhrevykh Teknology", Novosibirskaya obl. 630559 (RU)
(72) Inventor: RAMAZANOV, Yury Akhmetovich, Novosibirsk 630090 (RU); REPKOV, Andrei Petrovich, Novosibirskaya obl. 630559 (RU)
(74) Representative: Rupprecht, Kay
(86) International application number: PCT/RU2011/000612
(87) International publication number: WO 2013/025116

(57) **Abstract**

This invention relates to the apparatuses for carrying out biochemical processes using liquids of different viscosities, particularly when culturing the tissue cells and microorganisms in nutrient media of high viscosity, and can be used in various industries, particularly in biotechnology and food industry. The technical result of the invention is increased efficiency of stirring and accelerated biochemical processes in liquids of different viscosities. The bioreactor comprises a cylindrical tank (1) with a lid (2), disposed in a device for stirring a medium consisting of the bladed wheel (7) horizontally arranged on a vertical shaft in the upper part of the tank (1), and a horizontal annular baffle (9) arranged in the tank with a clearance with respect to the tank cylindrical wall, a rod (10) vertically mounted along the axis of the tank, a rotatable horizontal annular baffle (9), and a mechanism for fixation of the horizontal annular baffle (9) to the rod (10).

The bioreactor is provided with a pipe (19) or a telescopic pipe (20) fitting the axial opening (13) of the horizontal annular baffle (9). Said pipe is attached to the bottom of the annual baffle from below and arranged around the rod (10). There are radial channels (12) on the upper surface (11) of the horizontal annular baffle (9) passing from the axial opening (13) to the edge an annular baffle (9) being inclined downwards towards the bottom of the tank (1). The horizontal annular baffle (9) is configured floating as it is made of such material as, for example polypropylene. Inner diameter of the pipe (19) or telescopic tube (20) fits the diameter of the axial opening (13) of the horizontal annular baffle (9). The area of the axial opening (13) of the horizontal annular baffle (9) is equal to the total cross-sectional areas at the inlet (14) to the radial channels (12) and the total area of the outlet (15) of these radial channels (12).

## Description

### Technical Field

This invention relates to an apparatus for carrying out biochemical processes using fluids of different viscosity, particularly when culturing the tissue cells and microorganisms in nutrient media of high viscosity or liquids having a viscosity which changes (increases) during the mixing and can be used in various industries, particularly in biotechnology and food production.

### Background of the Invention

A bioreactor is disclosed in prior art. Said bioreactor comprises a cylindrical tank with a lid and pipes for the supply and removal of gas, and a device for aerating and mixing the medium. The device for aerating and stirring comprises a horizontal bladed wheel fixed to the vertical drive shaft and placed in the upper part of the tank directly below the lid. The device also comprises an annular plate located beneath the wheel; said plate having a central opening for the passage of gas is peripherally attached to the tank wall to form an annular cavity around the wheel for inflow and outflow of gas. There are slotted openings in an annular baffle formed for passage of gas. The slotted openings are arranged uniformly around the circumference obliquely to the horizontal plane (WO 92/05245).

The design of the bioreactor does not provide effective mixing of viscous culture medium in the biochemical processes. At high gas velocities, droplets with cultured cells are captured from the surface of the medium and thrown onto the tank wall resulting in injury and death of the cells.

Another bioreactor is disclosed in prior art. Said bioreactor comprises a cylindrical tank with a lid, a device for mixing a medium disposed in the tank, said device consisting of a bladed wheel mounted horizontally on a vertical shaft in the upper part of the tank, and an annular baffle arranged rotatable in the tank forming a gap with the tank wall and provided with floats. The bioreactor also comprises an annular baffle position adjusting mechanism for adjusting the height of the annular baffle relative to the stirred medium. This mechanism can contain racks attached to the tank lid and the annular baffle with clamps. Said racks allow changing the position of the baffle relative to height of the tank (RU 2099413 C1).

The disadvantage of the bioreactor is in that when the annular baffle is fixedly attached, large resistance to the movement of the viscous medium is created thereby impairing the process of mixing. Use of the floating annular baffle leads to its ejection onto the surface of the liquid and periodical vibration displacing it to the tank wall thereby deteriorating the mixing process.

The closest analog of the claimed invention is a bioreactor according to RU 2299903 C2, which comprises a cylindrical tank with a lid, a device for mixing a medium, said device comprising a bladed wheel horizontally fixed to a vertical shaft in the upper part of the tank, and an annular baffle rotatably arranged in the tank so that it forms a clearance with the tank wall. The baffle is provided with floats. The device also comprises a position adjusting mechanism for adjusting the annular baffle surface height relative to the stirred medium. This mechanism includes a vertical rod mounted along the axis of the tank, and a bushing attached to the rod and connected to the annular baffle by means of racks for its rotation round the rod. Said adjusting annular baffle position mechanism can be provided with a locking element to fix its position relative to the rod, said locking element comprising an additional bushing disposed between the rod and the bushing of the annular baffle, and the limiting stops of the baffle and at least one clamping element, wherein the rod is installed with the possibility of its axial displacement.

However, said reactor operates in liquid media having a viscosity not grater than 2 times the viscosity of water. When higher viscous media are used, the speed of the activator wheel should be significantly increased to create higher pressure over the surface of the stirred liquid. When this occurs, droplets with cultured cells are captured and thrown on the tank wall, resulting in cell injury and death. In addition, in such case in a viscous liquid the central up flow (axial counter flow) is not formed at the bottom of the reactor tank but somewhat higher, thereby a stagnant zone is formed.

Furthermore, when culturing plant and animal cells that are prone to agglomerate, especially in viscous media or in media with varying viscosity, there is the problem of cell or cell cluster settling on the upper surface of the annular baffle. In further development of anchored cell agglomerates death of cells in lower layers and poisoning of the environment by lysis products occur adversely affecting the results of the cultivation.

The technical result of the invention is to increase mixing efficiency and to accelerate biochemical processes in liquid media.

### Summary of the Invention

This result is achieved due to the claimed bioreactor comprising a cylindrical tank with a lid, a stirring device located in the tank, said stirring device consisting of a bladed wheel fixed horizontally on a vertical shaft in the upper part of the tank, and a horizontal annular baffle arranged in the tank with a gap relative to the cylindrical tank wall, a rod vertically mounted along the tank axis, wherein the rotatable horizontal annular baffle and the locking mechanism of the horizontal annular baffle are attached to said rod. According to the claimed invention, the bioreactor is provided with a pipe or a telescopic pipe fitting an axial opening of the horizontal annular baffle, attached to the baffle from below, and passing around the rod. The horizontal annular baffle is configured with radial channels passing from the axial opening to the edge of the annular baffle with a slope downwards towards the tank bottom.

A horizontal annular baffle is configured floating. It can be made for example of polypropylene. The inner diameter of the pipe or the telescopic pipe fits the diameter of the axial opening of the horizontal annular baffle.

The area of the axial opening of the horizontal annular baffle is equal to the total areas of cross sections at the inlets of the radial channels and the total cross-sectional areas at the outlet of the radial channels.

A mechanism adjusting position of the horizontal annular baffle comprises a bushing rotatably mounted on the rod above the horizontal annular baffle and attached to the latter by means of struts, and elements for limiting axial movement of the bushing along the rod.

The walls, the tank lid, and the horizontal annular baffle of the bioreactor can be made of an optically transparent material, for example of polycarbonate.

### Brief Description of the Drawings

The invention is illustrated by the following drawings.
Fig. 1 shows the basic layout of the bioreactor with a pipe on the annular baffle for mixing the medium of normal and high viscosity.
Fig. 2 shows a diagram of the bioreactor with the telescopic pipe on the annular baffle for stirring thick liquids.
Fig. 3 is a general view of the horizontal annular baffle with a pipe.
Fig. 4 is a diagram of a horizontal annular baffle, cross section along A-A.
Fig. 5 shows a diagram of a horizontal annular baffle, the top view.

### Description of Embodiments

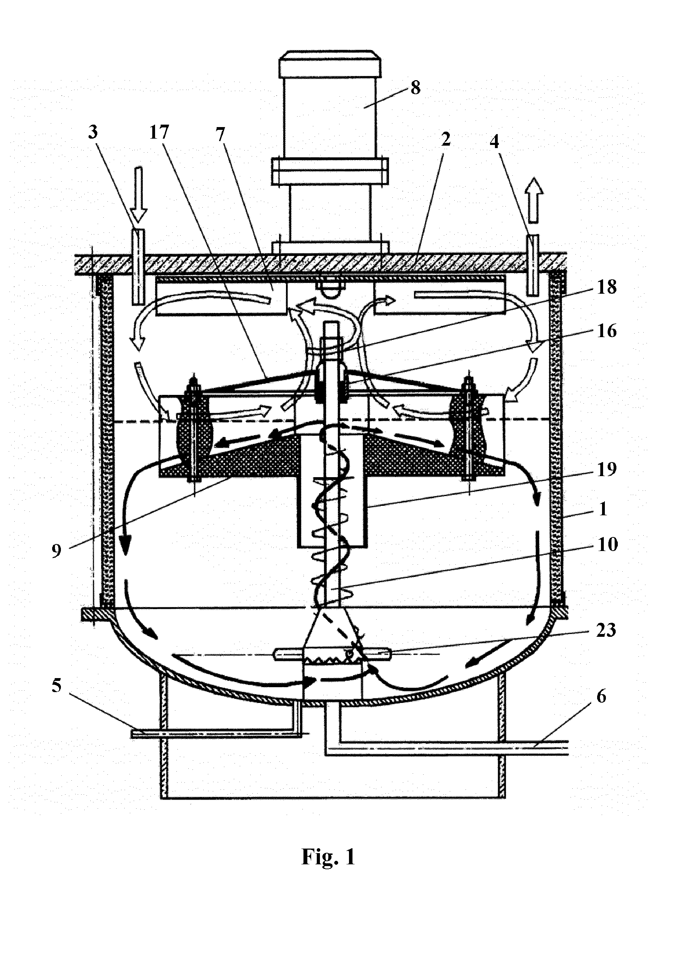

The bioreactor (Fig. 1) comprises a cylindrical tank 1 having a lid 2 with an inlet pipe 3 and an outlet pipe 4 for aerating gas, a feed pipe 5 for feeding initial components for cultivation and extracting the finished product, and an additional supply pipe 6 for the aerating gas. When photobacteria are cultured, the wall of the tank 1 and the lid 2 are made of an optically transparent material. A device for stirring a liquid medium is arranged inside the tank 1, said device comprising an activator bladed wheel 7 attached horizontally to a vertical shaft with a drive 8 located in the upper part of the tank 1, a horizontal annular baffle 9 mounted coaxially and rotatably in the tank 1, said baffle being arranged with a gap to the inner wall of the tank 1, and a rod 10 arranged vertically along the tank axis, said rod supporting mounted rotatable horizontal annular baffle 9. The lower end of the rod 10 is fixed at the bottom of the container 1.

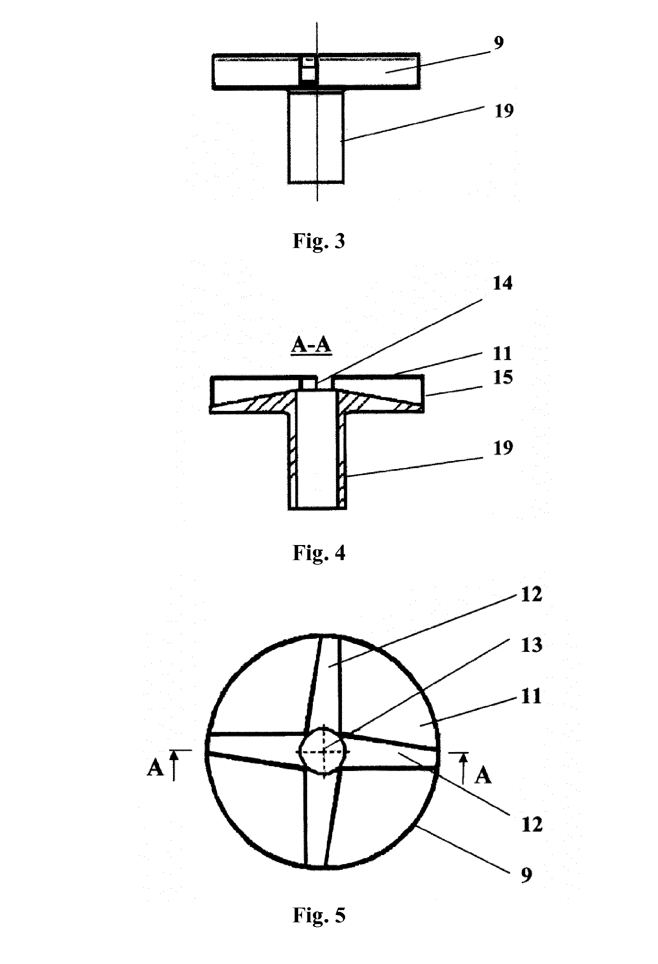

On the upper surface 11 of the horizontal annular baffle 9 (Figs. 3-5) there are radial channels 12 extending from the axial opening 13 to the edge of the annular baffle 9 with a slope downwards towards the bottom of the tank 1. The area of the axial opening 13 of the horizontal annular baffle 9 is equal to the total cross-section areas at the inlet 14 of the radial channels 12 and the total area of the outlet 15 of said radial channels 9.

Moreover, a horizontal annular baffle 9 can be floatable if it is made of such material as for example polypropylene.

The annular partition 9 is provided with a mechanism for adjusting its position on the rod 10, said mechanism comprises a bushing 16 mounted on the rod 10 above the horizontal annular partition 9 and is rotatably attached to the latter by means of struts 17, and the elements 18 for limiting axial movements of the bushing 16 along the rod 10. The bioreactor is provided with a pipe 19 for mixing and aerating liquids having normal viscosity (close to the viscosity of water) or a higher viscosity (Fig, 1). Said pipe 19 fits the axial opening 13 of the horizontal annular partition 9, and it is attached to the latter from below and around the rod 10.

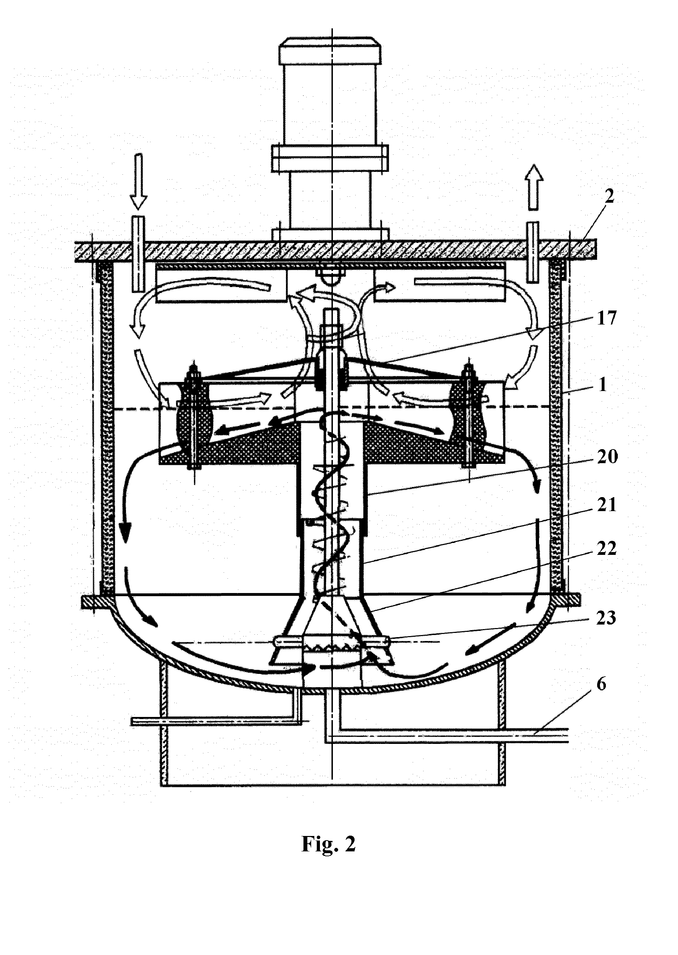

The bioreactor is provided with a telescopic pipe 20 for mixing and aerating liquids having high viscosity (Fig. 2). Said pipe has a lower element 21 with a socket 22. The lower element 21 with the socket 22 installed in the pipe 20 is configured for reciprocating movement. The pipe 6 for additional supply of aerating gas is connected to a perforated annular pipe 23 mounted in the axial zone at the bottom of the tank 1.

### Description of operation of the vertex Bioreactor

### Cultivation of bacteria, animal or plant cells

When culturing microorganisms such as bacteria or animal or plant cells, the cylindrical tank 1 with horizontal annular baffle 9 and pipe 19 attached to the rod 10 (Fig. 1) is filled under sterile conditions with the nutrient medium through the pipe 5 so that the surface of the medium in the upper part of the tank 1 leaves space for cavity for movement of the aerating gas, and the annular baffle 9 is disposed in a medium with the surface 13 above the liquid medium, as shown in Fig 1. Then the temperature is adjusted for culture, for example plant or animal cells, a seed cell dose is introduced through the pipe 5, and the actuator 8 of rotating the bladed wheel 7 is switched on. Depending on the requirements of the cultivation technology, a required speed of rotation of the bladed wheel 7 is set. During rotation of the wheel, the pressure decreases above the surface of the cell suspension in the axial area of the tank 1, and pressure increases at the periphery of the tank. Under the influence of the pressure difference between the axial zone and a peripheral zone in the gas cavity above the surface of the cell suspension, a swirling flow of gas forms, said flow generating turbulent rotary motion in the culture liquid resulting in intensive stirring along the axis of the tank. The annular baffle 9 with the pipe 19 rotates in the same direction and with the same angular velocity as the culture liquid, and it is held on the rod 10 due to its own buoyancy and by the hold elements 18. During culturing, the annular baffle 9 with the pipe 19 increases intensity of the liquid circulation (in the form of a tangential whirling motion with axial counterflow).The liquid medium with the microorganisms or cells of plants or animals in the form of the upstream rises through the pipe 19, the axial opening 13 of the annular baffle 9 and flows through the inclined channels 12 of the annular baffle 9 downwardly along the side walls of the cylindrical tank 1. The inclined radial channels 12 of the annular baffle 9 does not allow cell agglomerates settling on the surface of the baffle as they are thrown off the baffle 9 by the liquid flow. In the culturing process gas aerates cells or microorganisms on the surface of the liquid. As a result of the aeration, there are no gas bubbles and foam formed in the culture liquid, and cells and microorganisms are not damaged. The speed of movement of the gas vortex (3-6 m/s) is such that no culture liquid droplets come off the surface, which also reduces cell injury. Increasing upstream and downstream motion in the cell suspension allows culturing the cells without formation of stagnation zones when the height of the medium in the tank 1 is equal to or several times (2-3 times) bigger than the diameter of the tank 1. At the same time there are normal conditions for the cultivation of microorganisms. The resulting biomass is discharged through the pipe 5.

### Biotechnological processes with stirring highly viscous liquids in a vortex bioreactor.

When stirring liquids of high viscosity, for example for hydrolysis of starch for producing starch syrup, before starting the process, an annular baffle 9 with a telescopic pipe 20 is attached to the rod 10 (Fig. 2). The lower end of the telescopic pipe 20 extends close to the bottom of the tank 1 at the annular perforated pipe 23 for supplying additional aerating gas. Next, the tank 1 is filled with starch suspension through the pipe 5 so that the surface of the liquid in the upper part of the tank 1 allows a cavity for movement of gas, and the annular baffle 9 is disposed in a medium with the surface 13 above the surface of the liquid medium, as shown in Fig. 2. Then the temperature required for the hydrolysis of starch is adjusted and alpha-amylase enzyme is introduced into the medium through the pipe 5 and the rotary drive of the bladed wheel 6 is switched on. Depending on the technology requirements, the required number of revolutions of the bladed wheel 7 is set. Rotation of the bladed wheel over the surface of the starch suspension creates a vacuum in the axial zone of the tank 1 and increased pressure at the periphery of the tank. Under the pressure difference between the periphery and the axial zone of the gas cavity, a swirling gas flow is formed above the liquid surface, said flow creates turbulent rotary motion of the liquid with intensive stirring along the axis of the tank. When rotating the annular partition 9 with telescopic tube 20, which are rotated by the liquid flow in the same direction and with the same angular velocity and retained on the rod 10, providing an increase in intensity of upstream and downstream liquid flows. Due to the pressure difference between the periphery of the tank 1 (the pressure is higher at its sides) and the axial zone (reduced pressure), the axial upward flow of the viscous liquid passes into the telescopic pipe 20 through a gap between the bottom of the container 1 and the end of pipe 20 into the socket 21 of said pipe eliminating stagnant zones at the bottom of the tank 1. The centrifugal upflow of the liquid passes through the pipe 20, discharges through the axial opening 13 of the baffle 9 and flows further through the inclined radial channels 12 of the baffle 9 downwards along the cylindrical sidewalls of the tank 1. Inclined shape of the radial channels 12 of the annular baffle 9 does not allow thick starch suspension settling on the baffle surface as it is thrown off from said baffle 9 by the liquid flow. Next, the suspension moves down the periphery of the tank 1, forming a descending flow along the wall of said tank 1. Produced starch hydrolyzate is discharged through the pipe 5.

### Cultivation of microalgae or other photosynthetic microorganisms.

A bioreactor used for culturing photosynthetic microalgae has the cylindrical wall of the tank 1, the lid 2 and the horizontal annular baffle 9 made of an optically transparent material such as polycarbonate.

Light sources are mounted on the outside of the transparent lid 2 (not shown in the Figures). Under sterile conditions, the cylindrical tank 1 with the horizontal annular baffle 9 with the tube 19 attached to the rod 10 is filled wit the nutrient medium through the pipe 5 so that the surface of the medium in the upper part of the tank 1 leaves a cavity for the passage of the aerating gas, and the annular baffle 9 is located in a nutrient medium with the surface 13 above the liquid medium as shown in Fig .1. Then, for example, for culturing microalgae such as Chlorella, the required temperature is set, a seed dose of microalgae is introduced through the pipe 5 (e.g., Chlorella cells at 0.1 g per 1 liter of the culture medium), and the drive 8 of the bladed wheel 7 is switched on. Depending on the requirements of culturing technology, the number of revolutions of the bladed wheel 7 is set. Rotation of said wheel creates a decrease in pressure in the axial zone of the tank 1 and an increase in pressure on the periphery of the tank. Under the influence of the pressure difference between the peripheral zone and the axial zone of the gas cavity, a swirling flow of gas is created above the surface of the microalgae suspension, said flow forms turbulent rotary motion in the culture liquid providing intensive stirring along the axis of the tank.

The annular baffle 9 with the pipe 19 rotates in the same direction and with the same angular velocity as the culture liquid, and it is maintained at the rod 10 by its own buoyancy and the hold elements 18. During culturing, the annular baffle 9 with the pipe 19 increases the intensity of the liquid circulation (in the form of a tangential whirling motion with axial counterflow). The liquid medium with microalgae rises as upstream through the pipe 19 and the axial opening 13 of the baffle 9, and flows through the inclined radial channels 12 of the baffle 9 downwards along the cylindrical sidewalls of the tank 1. The inclined shape of the radial channels 12 of the annular baffle 9 does not allow microalgae settling on the surface of the baffle. Said microalgae are thrown off the baffle 9 by the liquid flow.

During the culturing, when applying the mixture of air and carbon dioxide through the feed pipe 6 for aerating gas and the perforated annular pipe 23, carbon dioxide is gripped by microalgae passing through the pipe 19 with the riser flow of the liquid. As a result of such aeration, the process of carbon dioxide consumption by microalgae, oxygen evolution, and breeding the culture are intensified. Absence of a traumatic stirring device inside the liquid allows cultivation of shell-less types of microalgae sensitive to mechanical stress. The speed of movement of the gas vortex (3-6 m/s) does not cause separation of culture liquid droplets from the surface, which also reduces injury of the microalgae. Adjusting intensity of upstream and downstream flows of the microalgae suspension using activator of the bladed wheel 7, it is possible to passing all liquid layers with microalgae under the light source providing required mode of consumption of light photons. Cultivation takes place without formation of dead zones when the height of the medium in the tank 1 is equal to or several times (2-3 times) bigger than the tank diameter, while providing optimal conditions for cultivation of all types of microalgae. The process is carried out while the biomass grows, for example of Chlorella algae, to 15-20 g per 1 liter of the suspension. The resulting biomass is discharged through the pipe 5.

Thus, the claimed bioreactor can effectively stir liquids of various viscosity including highly viscous liquids with simultaneous aeration without stagnant zones, and accelerate the biochemical processes in it.

## Claims

1. A vertex bioreactor comprising a cylindrical tank (1) with a lid (2), a device for stirring a medium, said device comprising a bladed wheel (7) mounted horizontally on a vertical shaft in the lower part of the tank, and a horizontal annular baffle (9) arranged in the tank with a clearness relative to the cylindrical wall of the tank; a rod (10) vertically arranged along the axis of the tank 1, said rod supporting the rotatable horizontal annular baffle (9) and a mechanism for adjusting position of the horizontal annular baffle (9) on the rod (10), **characterized in that** said bioreactor is provided with a pipe (19) or a telescopic pipe (20) fitting the axial opening (13) of the horizontal annular baffle (9), said pipe being attached to the baffle from below and arranged around the rod (10), and there are radial channels (12) on the upper surface (11) of the horizontal annular baffle (9) passing from the axial opening (13) to the edge of the horizontal annular baffle (9) and being inclined downwards to the tank (1) bottom.

2. The bioreactor according to claim 1, **characterized in that** the area of the axial opening (13) of the horizontal annular baffle (9) is equal to the total areas of cross sections at the input of the radial channels (12) and to the total area of the cross section at the output of the radial channels (12).

3. The bioreactor according to claim 1, **characterized in that** the horizontal annular baffle (9) is configured floating.

4. The bioreactor according to claim 1, **characterized in that** the inner diameter of the pipe (19) or the telescopic pipe (20) fits the diameter of the axial opening (13) of the horizontal annular baffle (9).

5. The bioreactor according to claim 1, **characterized in that** the mechanism for adjusting position of horizontal annular baffle (9) on the rod (10) comprises a rotatable bushing (16) placed on the rod (10) above the horizontal annular baffle (9) and attached to said baffle with struts (17), and elements (18) for limiting axial movements of the bushing (16) along the rod (10).

6. The bioreactor according to claim 1, **characterized in that** the lid (2) of the tank (1) and the horizontal annular baffle (9) of the bioreactor are made of optically transparent material.
